(19) Europäisches Patentamt
European Patent Office
Office européen des brevets

(11) Numéro de publication: **0 148 666**
**B1**

(12) **FASCICULE DE BREVET EUROPÉEN**

(45) Date de publication du fascicule du brevet: **20.05.87**

(21) Numéro de dépôt: **84402464.6**

(22) Date de dépôt: **30.11.84**

(51) Int. Cl.[4]: **C 07 C 59/245**, C 07 C 51/08, C 07 C 51/09, C 07 C 121/407, C 07 C 69/675

(54) **Procédé de préparation de l'acide hydroxy-3 méthyl-3 glutarique.**

(30) Priorité: **13.12.83 FR 8320313**

(43) Date de publication de la demande:
**17.07.85 Bulletin 85/29**

(45) Mention de la délivrance du brevet:
**20.05.87 Bulletin 87/21**

(84) Etats contractants désignés:
**AT BE CH DE FR GB IT LI LU NL SE**

(56) Documents cité:
**Néant**

(73) Titulaire: **SANOFI, 40, Avenue George V, F-75008 Paris (FR)**

(72) Inventeur: **Courregelongue, Jean, 3, rue George Clémenceau, 31120 Portet/Garonne (FR)**
Inventeur: **Lalo, Jack, 21, rue de la Digue, 31300 Toulouse (FR)**
Inventeur: **Maffrand, Jean- Pierre, 5, rue du Corps Franc Pommiès, 31120 Portet/Garonne (FR)**

(74) Mandataire: **Bressand, Georges, c/o CABINET LAVOIX 2 Place d'Estienne d'Orves, F-75441 Paris Cedex 09 (FR)**

**EP 0 148 666 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention est relative à un nouveau procédé de préparation de l'acide hydroxy-3 méthyl-3 glutarique (H.M.G.) de formule

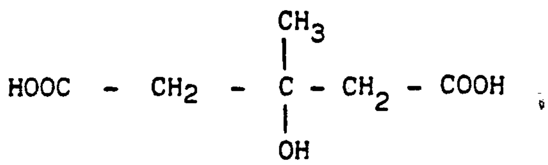

(I)

dont la dénomination commune internationale proposée est le MEGLUTOL. Sa biosynthèse hépatique est réalisée par l'enzyme H.M.C. CoA hydrolase (E.E. DEKKER, M.J. SCHLESINGER, M. COON; J. Biol. Chem., 1958, 233, 434). Le H.M.G. est donc un métabolite naturel du H.M.C. CoA, précurseur direct de la biosynthèse du cholesterol.

L'activité hypocholestérolémiante de l'acide hydroxy-3 méthyl-3 glutarique a été notamment mise en évidence chez l'animal par Z.H. BEG et M. SIDDIQUI (Experientia, 1967, 23, (5), 380) et chez l'homme par P.J. LUPIEN, D. BRUN et S. MOORJANI (Lancet, 1973, 1, 1256), et ce produit est actuellement commercialisé en Italie pour son activité hypolipémiante.

De nombreux procédés de préparation de ce composé ont été décrits dans la littérature:

- Oxydation du diallyl méthylcarbinol à l'aide d'ozone et de péroxyde d'hydrogène (H.J. KLOSTERMANN et F. SMITH, J. Amer. Chem. Soc., 1954, 76, 1129; Biochem. Preparations, 1958, 6, 25; R. TSCHECHE et H. MACHLEIDT, Justus Liebigs Ann. Chem, 1960, 631, 61)
- Réaction de Reformatsky entre l'acétoacétate d'éthyle et le bromoacétate d'éthyle (R. ADAMS et B.L. Van DUUREN, J. Amer. Chem. Soc, 1953, 75, 2377)
- Hydrolyse de l'hydroxy-3 méthyl-3 glutaronitrile (A.H. YAVROUIAN, R.A. SANCHEZ, J.K. POLLARD et E. METZNER, Synthesis, 1981, 10, 791)
- Oxydation permanganique du diallyl méthyl carbinol (Brevet IT. 030882 de la société AUSONIA)
- Oxydation permanganique du méthyl-3 pentanetriol-1,3,5 (Brevet E.P. 82 344 de E.K. METZNER).

Cependant tous ces procédés sont, soit difficilement transposables à l'échelle industrielle (réactions trop nombreuses, difficiles et dangereuses), soit trop onéreux (coût élevé des matières premières et/ou faibles rendements obtenus.).

La demanderesse a mis au point un procédé de synthèse peu onéreux, permettant d'obtenir l'acide hydroxy-3 méthyl-3 glutarique avec de bons rendements.

La présente invention a ainsi pour objet un procédé de préparation du composé de formule I décrite ci-dessus, caractérisé en ce que l'on condense le chloro-4 hydroxy-3 méthyl-3 butanoate d'éthyle de formule II suivante:

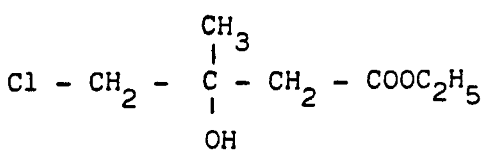

(II)

avec un cyanure de métal alcalin tel que le cyanure de sodium obtenant ainsi, le cyano-4 hydroxy-3 méthyl-3 butanoate d'éthyle de formule III suivante:

$$N \equiv C - CH_2 - \underset{\displaystyle OH}{\underset{|}{\overset{\displaystyle CH_3}{\overset{|}{C}}}} - CH_2 - COOC_2H_5 \qquad (III)$$

puis par hydrolyse, on obtient le composé de formule I.

La réaction de condensation est effectuée dans un solvant aprotique et polaire tel que par exemple le diméthyl sulfoxyde, à une température comprise entre 10° C et 140° C, et de préférence, entre 80° C et 100° C.

La réaction d'hydrolyse du composé de formule III s'effectue en milieu aqueux, en présence d'un hydroxyde de métal alcalin ou alcalinoterreux, de préférence l'hydroxyde de sodium, et de péroxyde d'hydrogène, à des températures comprises entre 20° C et la température d'ébullition.

Le composé chloro-4 hydroxy-3 méthyl-3 butanoate d'éthyle de formule II ci-dessus, est aisément obtenu par la réaction de condensation de Reformataky de la chloroacétone et du bromoacétate d'éthyle selon la méthode décrite par J. CURE et M. GAUDEMAR (Bull. Soc. Chim. France, 1969, 7, 2471-6).

Le procédé de l'invention permet donc, en 3 étapes aisément réalisables et avec des matières de départ peu coûteuses et facilement accessibles, d'effectuer la préparation du composé de l'invention avec un rendement global de l'ordre de 36 p. 100.

**Les exemples suivants illustrent l'invention**

1°) Préparation du chloro-4 hydroxy-3 méthyl-3 butanoate d'éthyle (II):

Dans une suspension de 44 g (0,67 atome-gramme; 1,87 équivalent) de zinc en poudre dans 30 ml d'éther diéthylique, on verse à température ambiante, sous agitation et sous atmosphère inerte, une petite quantité d'une solution renfermant 33 g (0,36 mole) de chloroacétone et 62 g (0,37 mole; 1,03 équivalent) de bromoacétate d'éthyle dans un mélange de 80 ml de benzène et 80 ml d'éther diéthylique, pour amorcer la réaction.

On ajoute ensuite le reste de la solution en un goutte à goutte rapide, pour assurer le reflux qui est maintenu par chauffage pendant deux heures après la fin de l'addition.

On refroidit ensuite le milieu réactionnel, acidifie par l'acide chlorhydrique N et filtre pour éliminer les sels minéraux. On décante la phase organique qu'on lave à l'eau et sèche sur du sulfate de sodium anhydre.

Par évaporation à sec, on recueille un résidu que l'on purifie par distillation sous pression réduite.

On obtient avec un rendement de 78 p. 100, une huile incolore, dont le point d'ébullition (0,01) est de 43° C.

2°) Préparation du cyano-4 hydroxy-3 méthyl-3 butanoate d'éthyle (III)

On verse goutte à goutte, à une température de 85°-90° C, sous atmosphère inerte, 71,6 g (0,39 mole) de chloro-4 hydroxy-3 méthyl-3 butanoate d'éthyle (II), dans une solution de 23,2 g (0,47 mole) de cyanure de sodium dans 150 ml de diméthylsulfoxyde et on chauffe à 100° C pendant 3 heures.

Le milieu réactionnel est ensuite versé sur un mélange de glace pilée et d'une solution aqueuse d'hydroxyde de sodium 2 N, puis on extrait à l'aide de dichlorométhane.

La phase organique est lavée à l'eau et séchée sur du sulfate de sodium anhydre. L'évaporation à sec laisse un résidu qui est purifié par distillation.

On recueille avec un rendement de 71 p. 100, une huile incolore dont le point d'ébullition (0,01) est de 89° C.

3°) Préparation de l'acide hydroxy-3 méthyl-3 glutarique (I)

On verse goutte à goutte à 0° C, sous atmosphère inerte, 50 ml d'une solution aqueuse d'eau oxygénée à 30 p. 100, dans un mélange de 20 g (0,12 mole) de cyano-4 hydroxy-3 méthyl-3 butanoate d'éthyle (III), 300 ml d'eau et 44 ml d'une solution aqueuse à 50 p. 100 d'hydroxyde de sodium.

On laisse le milieu réactionnel pendant 12 heures à la température ambiante, puis on chauffe progressivement jusqu'à léger reflux, que l'on maintient pendant 6 heures.

Après acidification par de l'acide chlorhydrique 12 N, et évaporation à sec, le résidu est repris par de l'acétone, filtré, évaporé à sec et purifié par recristallisation dans l'acétate d'éthyle.

On obtient, avec un rendement de 64,5 p.100, des cristaux blancs, dont le point de fusion est de 108° C

**Revendications**

pour les Etats Contractants: BE, CH, DE, FR, GB, IT, LI, LU, NL, SE

1 - Procédé de préparation de l'acide hydroxy-3 méthyl-3 glutarique de formule

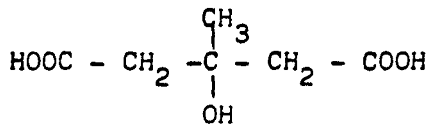

(I)

caractérisé en ce que l'on condense le chloro-4 hydroxy-3 méthyl-3 butanoate d'éthyle de formule II suivante

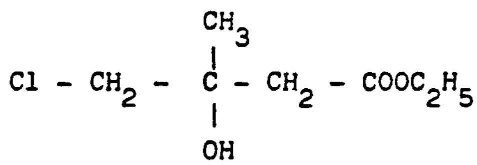

(II)

avec un cyanure de métal alcalin pour obtenir le cyano-4 hydroxy-3 méthyl-3 butanoate d'éthyle de formule III suivante

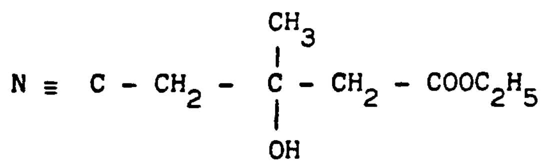

(III)

qui, par hydrolyse, fournit le composé de formule I.

2 - Procédé selon la revendication 1 caractérisé en ce que la réaction de condensation est effectuée dans un solvant aprotique et polaire tel que le diméthyl sulfoxyde.

3 - Procédé selon la revendication 2 caractérisé en ce que la réaction de condensation est réalisée à une température comprise entre 10° C et 140° C, et de préférence, entre 80° C et 100° C.

4 - Procédé selon la revendication 1 caractérisé en ce que l'hydrolyse du composé de formule (III) s'effectue en milieu aqueux, en présence d'un hydroxyde de métal alcalin ou alcalino-terreux tel que l'hydroxyde de sodium et de péroxyde d'hydrogène.

5 - Procédé selon la revendication 4 caractérisé en ce que l'hydrolyse est réalisée à des températures comprises entre 20° C et la température d'ébullition.

6 - Le cyano-4 hydroxy-3 méthyl-3 butanoate d'éthyle de formule III, composé nouveau utile pour la mise en oeuvre du procédé selon la revendication 1.

**Revendications**

pour l'Etat contractant AT

1 - Procédé de préparation de l'acide hydroxy-3 méthyl-3 glutarique de formule

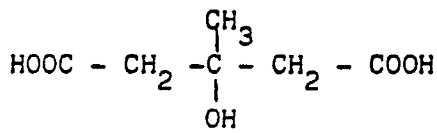

(I)

caractérisé en ce que l'on condense le chloro-4 hydroxy-3 méthyl-3 butanoate d'éthyle de formule II suivante

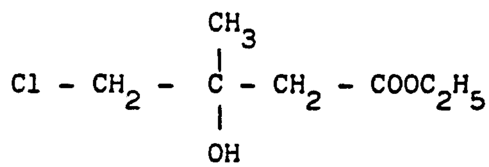

(II)

avec un cyanure de métal alcalin pour obtenir le cyano-4 hydroxy-3 méthyl-3 butanoate d'éthyle de formule III suivante

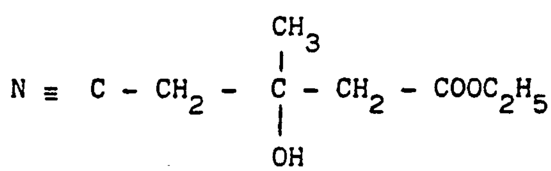

(III)

qui, par hydrolyse, fournit le composé de formule I.

2 - Procédé selon la revendication 1 caractérisé en ce que la réaction de condensation est effectuée dans un solvant aprotique et polaire tel que le diméthyl sulfoxyde.

3 - Procédé selon la revendication 2 caractérisé en ce que la réaction de condensation est réalisée à une température comprise entre 10°C et 140°C, et de préférence, entre 80°C et 100°C.

4 - Procédé selon la revendication 1 caractérisé en ce que l'hydrolyse du composé de formule (III) s'effectue en milieu aqueux, en présence d'un hydroxyde de métal alcalin ou alcalino-terreux tel que l'hydroxyde de sodium et de péroxyde d'hydrogène.

5 - Procédé selon la revendication 4 caractérisé en ce que l'hydrolyse est réalisée à des températures comprises entre 20°C et la température d'ébullition.

**Patentansprüche**

für die Vertragsstaaten: DE, CH, DE, FR, GB, IT, LI, LU, NL, SE

1. Verfahren zur Herstellung von 3-Hydroxy-3-methyl-glutarsäure der Formel

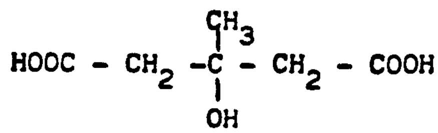

(I)

dadurch gekennzeichnet, daß man 4-Chloro-3-hydroxy-3-methyl-buttersäureethylester der folgenden Formel II

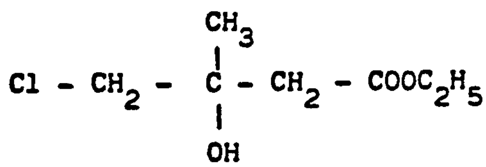

(II)

mit einem Alkalimetallcyanid kondensiert, um den 4-Cyano-3-hydroxy-3-methyl-buttersäureethylester der folgenden Formel III

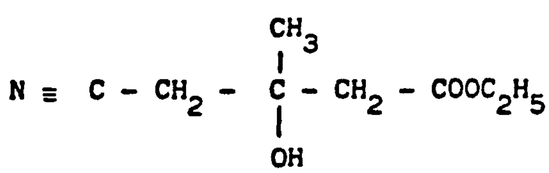

(III)

zu erhalten, der durch Hydrolyse die Verbindung der Formel I ergibt.

2. Verfahren nach Anspruch 1, dadurch gekennzeichnet, daß die Kondensationsreaktion in einem aprotischen und polaren Lösungsmittel, wie dem Dimethylsulfoxid, durchgeführt wird.

3. Verfahren nach Anspruch 2, dadurch gekennzeichnet, daß die Kondensationsreaktion bei einer Temperatur zwischen 10°C und 140°C, vorzugsweise zwischen 80°C und 100°C, durchgeführt wird.

4. Verfahren nach Anspruch 1, dadurch gekennzeichnet, daß die Hydrolyse der Verbindung der Formel (III) in wäßrigem Milieu in Gegenwart eines Alkalimetallhydroxids oder Erdalkalimetallhydroxids, wie Natriumhydroxid, und von Wasserstoffperoxid durchgeführt wird.

5. Verfahren nach Anspruch 4, dadurch gekennzeichnet, daß die Hydrolyse bei Temperaturen zwischen 20 -C und der Siedetemperatur durchgeführt wird.

6. 4-Cyano-3-hydroxy-3-methyl-buttersäureethylester der Formel III, ein neues Produkt, das für die Durchführung des Verfahrens gemäß Anspruch 1 verwendbar ist.

**Patentansprüche**

für den Vertragsstaat: AT

1. Verfahren zur Herstellung von 3-Hydroxy-3-methyl-glutarsäure der Formel

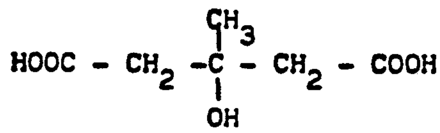

(I)

dadurch gekennzeichnet, daß man 4-Chloro-3-hydroxy-3-methyl-buttersäureethylester der folgenden Formel II

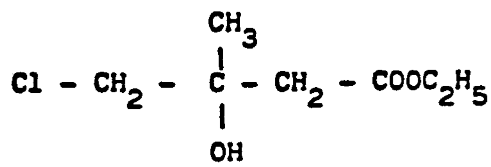

(II)

mit einem Alkalimetallcyanid kondensiert, um den 4-Cyano-3-hydroxy-3-methyl-buttersäureethylester der folgenden Formel III

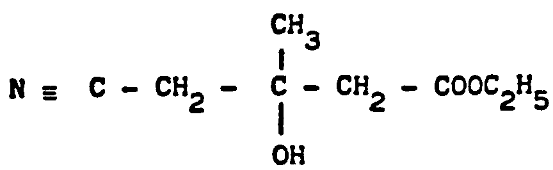

(III)

zu erhalten, der durch Hydrolyse die Verbindung der Formel I ergibt.

2. Verfahren nach Anspruch 1, dadurch gekennzeichnet, daß die Kondensationsreaktion in einem aprotischen und polaren Lösungsmittel, wie dem Dimethylsulfoxid, durchgeführt wird.

3. Verfahren nach Anspruch 2, dadurch gekennzeichnet, daß die Kondensationsreaktion bei einer Temperatur zwischen 10° C und 140° C, vorzugsweise zwischen 80 -C und 100° C, durchgeführt wird.

4. Verfahren nach Anspruch 1, dadurch gekennzeichnet, daß die Hydrolyse der Verbindung der Formel (III) in wäßrigem Milieu in Gegenwart eines Alkalimetallhydroxids oder Erdalkalimetallhydroxids, wie Natriumhydroxid, und von Wasserstoffperoxid durchgeführt wird.

5. Verfahren nach Anspruch 4, dadurch gekennzeichnet, daß die Hydrolyse bei Temperaturen zwischen 20° C und der Siedetemperatur durchgeführt wird.

**Claims**

for the Contracting States: BE, CH, DE, FR, GB, IT, LI, LU, NL, SE

1. Process for preparing 3-hydroxy 3-methyl glutaric acid having the formula

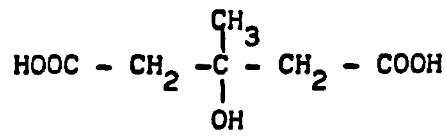

(I)

characterized in that ethyl 4-Chloro 3-hydroxy 3-methyl-butanoate of the following formula II

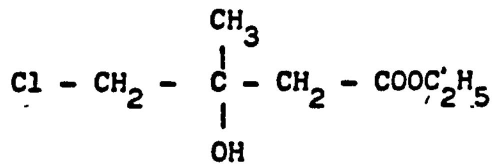

(II)

is condensed with an alkaline metal cyanide so as to obtain ethyl 4-cyano 3-hydroxy 3-nethyl butanoate of the following formula III

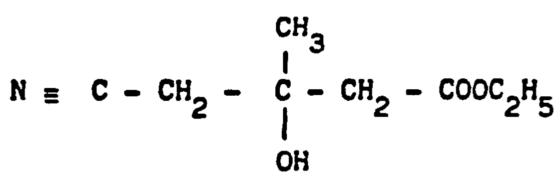

(III)

which by hydrolysis gives the compound of formula I.

2. Process according to claim 1 characterized in that the condensation reaction is carried out in an aprotic and polar solvent such as dimethylsulfoxide.

3. Process according to claim 2 characterized in that the condensation reaction is carried out at a temperature comprised between 10° C and 140° C and preferably between 80° C and 100° C.

4. Process according to claim 1, characterized in that the hydrolysis of the compound of formula (III) is carried out in aqueous medium, in the presence of an alkaline or alkaline-earth metal hydroxide such as sodium hydroxide and of hydrogen peroxide.

5. Process according to claim 4, characterized in that the hydrolysis is carried out at temperatures comprised between 20° C and the boiling temperature.

6. Ethyl 4-cyano 3-hydroxy 3-methyl butanoate of formula III, a new compound useful for carrying out the process according to claim 1.

**Claims**

for the contracting state AT

1. Process for preparing 3-hydroxy 3-methyl glutaric acid having the formula

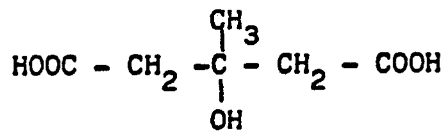

(I)

characterized in that ethyl 4-chloro 3-hydroxy 3-methyl-butanoate of the following formula II

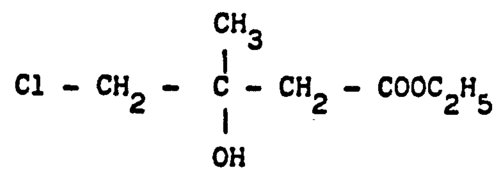

(II)

is condensed with an alkaline metal cyanide so as to obtain ethyl 4-cyano 3-hydroxy 3-methyl butanoate of the following formula III

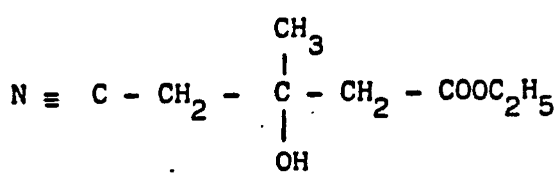

(III)

which, by hydrolysis, gives the compound of formula I.

2. Process according to claim 1, characterized in that the condensation reaction is carried out in an aprotic and polar solvent such as dimethylsulfoxide.

3. Process according to claim 2, characterized in that the condensation reaction is carried out at a temperature comprised between 10° C and 140° C and preferably between 80° C and 100° C.

4. Process according to claim 1 characterized in that the hydrolysis of the compound of formula (III) is carried out in aqueous medium, in the presence of an alkaline or alkaline-earth metal hydroxide such as sodium hydroxide and of hydrogen peroxide.

5. Process according to claim 4, characterized in that the hydrolysis is carried out at temperatures comprised between 20° C and the boiling temperature.